# EUROPEAN PATENT SPECIFICATION

(11) **EP 2 802 317 B1**
(45) Date of publication and mention of the grant of the patent: **09.09.2015**
(21) Application number: 13703726.3
(22) Date of filing: 10.01.2013
(51) Int. Cl.: A61K 9/70, B32B 7/06, A61F 13/00, B65D 75/30

(54) **PACKAGING STRUCTURE FOR BIOMEDICAL FILMS**
VERPACKUNGSSTRUKTUR FÜR BIOMEDIZINISCHE FILME
STRUCTURE D'EMBALLAGE DE FILMS DANS LE DOMAINE BIOMÉDICAL.

(30) Priority: 13.01.2012 IT MI20120031
(43) Date of publication of application: 19.11.2014
(73) Proprietor: Altergon S.A., 6900 Lugano (CH)
(72) Inventor: DI GRIGOLI, Maurizio, 20099 Sesto S. Giovanni (MI) (IT); COMUZIO, Sergio, 20099 Sesto S. Giovanni (MI) (IT)
(74) Representative: Gerli, Paolo
(86) International application number: PCT/EP2013/000049
(87) International publication number: WO 2013/104537

(56) References cited:
- EP-B1- 1 996 172
- US-A1- 2003 113 492
- US-A1- 2012 006 710

## Description

### FIELD OF THE INVENTION

The present invention relates to the packaging of therapeutic products, specifically biomedical films, and to methods for the production of said packages. A new packaging structure for biomedical films, particularly suitable for protecting the film and for manufacturing low-cost packages, is described.

### PRIOR ART

Biomedical films are thin laminar structures which are used for therapeutic and/or cosmetic purposes and are intended to administer active principles or in any case to be placed in contact with the tissue of the human or animal body. They are made of a matrix of thin, biocompatible and optionally bio-adhesive material. They may contain, dispersed in the matrix of the film, active substances intended for local or systemic administration, depending on the application site. Biomedical films are generally placed in contact with soft tissues such as mucous membranes or surfaces of internal organs; they may however also be used for superficial topical applications, for example on skin which is intact or skin which is affected by injuries or skin diseases. When designed for oral administration, they may have rapid-disintegration characteristics such that, when they come into contact with saliva, they disintegrate rapidly and release the active principle contained therein; the active principle thus released may be absorbed by the oral, sublingual or gastrointestinal mucosa.

Biomedical films may have various functions, for example a protective, repairing and locally curing function, or, when suitably charged with an active principle, they are applied onto highly vascularized mucous membranes, with the aim of releasing the active principle systemically or locally. These forms constitute a useful alternative to the traditional systemic administration methods such as oral, intravenous, intramuscular, etc. Films containing active principles may also be used for local treatment of the said mucous membrane or organ to which they are applied; depending on the structure of the matrix, the film may also act as a support for the controlled and prolonged release of the active principle. Rapidly disintegrating biomedical films may be used for oral administration: once placed in the mouth, they dissolve rapidly before being swallowed, allowing gastrointestinal absorption of the active principles.

Common characteristics of biomedical films are the small thickness and the high flexibility in order to ensure user compliance. Consequently, these products are extremely delicate and prone to breakage or damage, in particular during production of the package, or opening thereof by the patient, or during removal and application.

The packages of biomedical films may consist of two thin plastic laminates overlapping each other and heat-sealed along their external perimeter so as to form a sealed-envelope structure housing the film inside the two laminates. The heat-sealable material of the laminate is made of polyethylene or other similar material; the laminate often comprises further overlapped layers with insulating/reinforcing function (for example metal foil) and, externally thereto, a printable support layer (e.g. paper), etc.

The packaging method described above involves various problems, in particular: the risk of damaging the film by the heat applied during sealing of the package; the risk, in the event of machine stoppage, of burning of the packaging material present in the proximity of the sealing station; the need to provide a sufficiently broad edge of the package which is to be heat-sealed, with a consequent increase in the size of the package and the material required to produce it. There therefore exists the need for new packaging structures which are more effective and suitable for biomedical films, in particular oral films.

### DESCRIPTION OF THE FIGURES

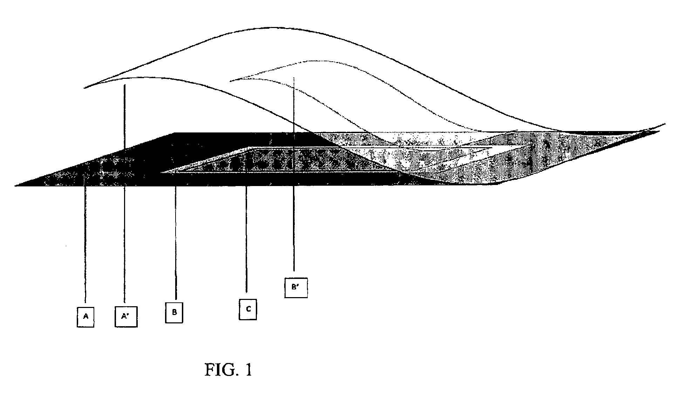

Fig.1 shows a three-dimensional illustration of the packaging structure according to the invention.

### SUMMARY

A new form of packaging for biomedical films, which totally eliminates the problems associated with conventional packaging, has now been found and forms the subject of the present invention. With reference to Figure 1, the new form of packaging is a multilayer structure comprising the following layers, overlapped in the following order:
- (A) a laminate;
- (B) a supporting layer;
- (C) a biomedical film;
- (B') a supporting layer;
- (A') a laminate;
wherein:
- the laminates (A) and (A') reversibly adhere to each other in the cold state,
- the said supporting layers are smaller in area than the said laminates and are positioned fully within the laminate area, thereby determining a perimetral band (P) in which the internal faces of the laminates (A) and (A') are in reversible adhesive contact with each other.

The adhesive contact between (A) and (A') in the perimetral band P isolates completely the supporting layers from the external environment; the supporting layers define, by means of their respective opposite internal surfaces, a non-adhesive receiving seat inside which the film (C) is present. This receiving seat is completely isolated from the external environment, owing to the adhesion between (A) and (A') along the perimetral band P.

### DETAILED DESCRIPTION

The terms "external" and "internal" used herein are referred to the position of the film (C); "internal" is what is directed towards the film (C); "external" is what is directed in the direction opposite to the film (C).

The laminates (A) and (A') perform the function of a protective external structure. The term "cold adhesive", relating to the laminate (A), indicates that the internal face of this laminate has adhesive characteristics such as to allow adhesion thereof to the internal face of (A'), at room temperature, by means of simple contact and without the need for heat application. Similarly, the internal face of (A') has adhesive characteristics such as to allow adhesion thereof to the internal face of (A), at room temperature, by means of simple contact and without the need for heat application. The assembly of the laminates (A) and (A') thus differs from heat-sealable materials which do not adhere to each other at room temperature.

The aforementioned adhesive characteristics may be present on both the internal faces of (A) and (A'), or on a single face thereof, provided that the adhesiveness of one face is sufficient to ensure stable adhesion with the other face.

The aforementioned conditions may be satisfied by adhesive laminates or by laminates which are originally not adhesive, but are able to develop sufficient adhesiveness through contact, where necessary assisted by the application of a suitable pressing force, at room temperature.

The term "at room temperature" identifies a temperature range of between about 10°C and 40°C, preferably between 15°C and 30°C, and even more preferably between 20°C and 25°C. The laminates (A) and (A') thus adhere together without the need for heat-sealing. It is understood, however, that the adhesiveness developed at room temperature is maintained also at temperatures which exceed said range, on average by +/- 15°C, such that the adhesion at room temperature remains stable also in the case of high temperature fluctuations.

The adhesion between (A) and (A') is reversible such that the two laminates may be subsequently separated from each other by means of application of a suitable pulling force by the user.

The laminates (A) and (A') may be made using common supporting materials, the internal face of which is coated with adhesive material. In addition to the adhesiveness of the internal face, the laminate may comprise further layers with specific functions, for example a metal foil with a reinforcing and/or isolating function, a sheet of paper acting as a printable support, an external plastic material providing a finish, etc.

The supporting layers (B) and (B') (which may also be defined as "liners" for the purposes of the present application) are laminar supports without adhesive properties, at least on their internal face, i.e. their face situated respectively opposite to the laminates (A) and (A'). In the present structure they are arranged mutually overlapped, forming with their opposite internal faces a suitable receiving seat for the film (C) and ensuring that the latter is isolated from the adhesive internal surface of the laminate (A); without the supporting layer, in fact, the film would come into direct contact with the adhesive side of the laminate (A), remaining glued thereto and being no longer removable. The supporting layer also acts as a support for spreading the film, during the production process described further below. Finally, the supporting layer constitutes a further element for isolating/protecting the film, which is not present in the known packages, being advantageously centred on the position occupied by the film.

On the external side, the supporting layers (B) and (B') come into contact with the adhesive internal face of the respective external laminates (A) and (A'), therefore adhering thereto. It is not necessary for the supporting layers to be adhesive per se on the external face, although this variant is included within the present invention; when the supporting layer is externally provided with own adhesiveness, this may be substantially of any type; preferably, but not exclusively, it consists of cold adhesiveness.

The supporting layers may be made using any material which ensures complete isolation of the film (C) from the adhesive material present on the external laminate, also maintaining a suitable impermeability, such that the film contained between (B) and (B') maintains unchanged its original degree of moisture. The supporting layer also has mechanical characteristics sufficient for allowing the spreading thereon of the film precursor in liquid form; optionally, moreover, it may have structural reinforcing characteristics for additional protection of the film inside the final package. Typically, the supporting layer may consist of polyester, laminated paper, and similar materials with characteristics such as to ensure easy separation of (C) at the time of use.

The film (C) may be any biomedical film. Such films may include oral films, e.g. of the rapidly disintegrating or muco-adhesive type, with or without active principles dispersed in the matrix. The matrix of the film may consist of biocompatible and optionally biodegradable materials such as cellulose derivatives, hyaluronic acid, chitosan, modified starches, gums, polylactide/polyglycolide copolymers, synthetic polymers such as polyacrylates, polyamides, polyethylene, polyethylene glycols, polyvinylpyrrolidone, etc. Said films may be bioadhesive, namely able to adhere by means of contact to a target tissue (mucous membranes, skin, etc.).

The active principle, when present in the film, may be any compound with a therapeutic activity, to be administered locally or systemically.

The present package can be opened by the user by taking advantage of the reversibility of adhesion between (A) and (A'): the two laminates can thus be separated from each other, without tearing, by applying a suitable pulling force to one of them. The separating action may be rendered easier by providing, on the two laminates, non-adhesive perimetral zones (edge portions) on which the separating stress is preferably applied; in another embodiment, one of the two laminates may extend beyond the area of the facing laminate, thus forming a superficial discontinuity (step) on which the separating stress is preferably applied; other embodiments are possible by employing alternative solutions which are known per se.

During separation, each laminate pulls along with it the supporting layer adhering to its internal face; in this way the receiving seat inside which the film is stored opens and the user may remove the film; removal is easy since the internal faces of the supporting layers, in contact with the film, are not adhesive.

The technical solution described above offers numerous advantages. Firstly, owing to the cold adhesiveness, it is possible to carry out the entire packaging cycle at room temperature, without applying heat produced by heat-sealing which could damage the outer edges of the film. Moreover, with cold processing, it is possible to interrupt at any time the packaging cycle without subjecting the material which is at a standstill in the sealing station to the risk of burning. Finally, with the invention it is possible to provide packages with a smaller surface area, given the same extension of the film; in fact, in the case of conventional heat-sealing, it was required to provide a relatively broad, outer, perimetral band inside which the outer edges of the film could be spaced from the heat-sealing line; this requirement is no longer necessary in the present invention: here the film may be housed much closer to the point of adhesion of the two external laminates, without the need to provide a separation zone. The smaller packaging area results in saving the amount of packaging material and greater compactness of the packages.

Moreover, the supporting layer constitutes a further reinforcing/isolating layer which is advantageously centred on the surface of the film: it is therefore possible to reduce the isolating requisites of the external laminate which may therefore be a material which is constructionally more simple/thinner than those commonly used, and therefore less expensive, without adversely affecting isolation of the packaged film.

The process for preparing the packaging according to the invention comprises assembling the components (A), (B), (C), (B'), (A') as defined above and in the order previously described.

In a preferred production method, production comprises the following steps:
1. providing a first laminate strip;
2. intermittently applying a supporting layer on the laminate obtained in step 1);
3. applying the film on the supporting layer present on the laminate obtained in step 2);
4. providing a second laminate strip as obtained in steps 1 and 2;
5. overlapping the laminate obtained in step 3) with the laminate obtained in step 4);
6. marking and/or cutting the composite laminate obtained in step 5), in the areas separating each supporting layer from the next one.

In step 1), the laminate is provided, as commercially available, without particular pre-treatment, in sizes suitable for the production line.

In step 2), the supporting layer is intermittently applied, for example by means of a suitable punch or similar system, which acts with a regular frequency on a laminate strip which is fed at a constant speed. In a similar manner the strip described in step 4) is produced.

The laminate strip obtained in steps 2) and 4) may be in the form of a single strip containing a single line of supporting layers arranged intermittently; this strip may be used directly during the following steps or stored as a semi-finished product, in the form of a wound single strip; however, for production purposes, the strips described in steps 2) and 4), preferably have a greater width, such as to form a sheet, onto which are applied, in parallel, n lines of supporting layers arranged intermittently; in this case the wound semi-finished product assumes the form of a roll which will be subsequently cut into n transverse sections, thus forming n + 1 single strips.

In step 3), application of the film is performed using techniques which are conventional per se, preferably by spreading a suitable liquid solution acting as precursor for the film: upon subsequent partial drying, the film assumes the desired mechanical characteristics; other deposition methods alternative to spreading are possible, for example spraying, injection, hot melting, etc. Application of the film is performed on the previously described strip, which is preferably fed at a constant speed inside a suitable film deposition station where the supporting layer is identified, for example using optical systems, and the liquid solution of the film is dispensed thereon; the film then passes into a suitable drying station where it assumes the required mechanical characteristics.

In step 5), the laminates obtained in steps 3) and 4), for example unwound from suitable strips, are adhesively joined together, on the respective internal faces, so that the supporting layers present thereon are overlapped on each other. Adhesion of the laminates may be promoted by pressing, for example by passing the obtained composite laminate through suitable rolls. Adhesion between the two laminates in any case takes place at room temperature, without the need to apply heat.

In step 6), the composite laminate obtained is cut along the transverse sections which separate one supporting layer from the next one. It is thus possible to obtain final packages, each containing a single film housed and protected between the two overlapped supporting layers which are in turn contained and isolated from the outside by the two external laminates, adhesively joined together along the perimetral band P. For the purposes of the invention is not indispensable to separate from each other all the films contained in a strip. For example it is possible to produce small-length strips containing a given number of films, for example to cover a complete treatment cycle; these strips are supplied to the user, suitably provided with cutting lines which are marked or printed between one film and the next one, or being provided in these positions with pre-cut lines; in such cases the user separates the single films, thereby producing the single package at the time of use.

In an alternative production method, the processing operations according to steps 1, 2, 4, 5 and 6 may be performed so as to obtain a film-free composite laminate, to be used immediately for application of the film or to be stored as a wound, single, composite strip (or composite roll to be cut into n single strips, as described above). During application of the film, taking advantage of the reversibility of adhesion between (A) and (A'), the composite strip is temporarily opened by suitable means provided for this purpose; the film as described in step 3) is deposited on one of the two laminates thus opened; this is then followed by steps 4) to 6) as described above.

In a further alternative production method, the film (C) is first applied onto the supporting layer (B); then the system (B)+(C) is combined with the remaining components of the packaging (A), (B'), (A') by means of the previously described production methods, suitably adapted, thus forming the packaging structure according to the invention.

In a still further alternative production method, the film (C) is first applied onto the supporting layer (B) and covered with the supporting layer (B'); then the system (B)+(C)+(B') is combined with the remaining components of the packaging (A), (A') by means of the previously described production methods, suitably adapted, thus forming the packaging structure according to the invention.

The finished packaging structure may be provided with an external wrapping and supplied to the patient in single form for extemporaneous treatment; or a container with a plurality of packaged films (separated or in the form of a sub-strip), for repeated treatment, for example covering one or more treatment cycles, may be provided. These external wrappings / containers also contain a suitable illustrative leaflet with the necessary information regarding the pharmacological action of the product, the application site and mode of application of the film.

The present invention also embraces the use of a structure comprising the elements (A), (B), (B'), (A') described above, overlapped on each other in the order indicated, for packaging a biomedical film.

## Claims

1. Packaging structure of biomedical films comprising the following overlapped layers, in the following order:
- (A) a laminate;
- (B) a supporting layer;
- (C) a biomedical film;
- (B') a supporting layer;
- (A') a laminate;
wherein:
- the laminates (A) and (A') reversibly adhere to each other at room temperature
- the said supporting layers are smaller in area than the said laminates and are positioned fully within the laminate area, thereby determining a perimetral band P in which the internal faces of the laminates (A) and (A') are in reversible adhesive contact with each other.

2. Structure according to claim 1, wherein both laminates (A) and (A') are adhesive on the respective internal faces.

3. Structure according to claim 1, wherein one of the two laminates (A) or (A') is adhesive on its internal face.

4. Structure according to claims 1-3, wherein the laminates (A) and/or (A') are multilayer structures.

5. Structure according to claims 1-4, wherein the supporting layers (B) and/or (B') are made of laminated paper, or polyester.

6. Structure according to claims 1-5, wherein the supporting layers (B) and/or (B') are adhesive on their external face.

7. Structure according to claims 1-6, wherein the biomedical film is a film for oral application.

8. Structure according to claims 1-7, in the form of a multi-use strip containing a sequence of oral films, separated from each other by marking lines.

9. Process for the preparation of a packaging structure for biomedical films, comprising assembling the components (A), (B), (C), (B'), (A') as defined in claim 1 and in the order indicated therein.

10. Process according to claim 9, comprising the following steps:
1. providing a first laminate strip;
2. intermittently applying a supporting layer on the laminate obtained in step 1;
3. applying a film on the supporting layer present on the laminate obtained in step 2;
4. providing a second laminate strip according to steps 1 and 2;
5. overlapping the laminate obtained in step 3 with the one obtained in step 4;
6. marking and/or cutting the composite laminate obtained in step 5, in the areas separating each supporting layer from the next one.

11. Process according to claim 9, wherein the overlapped layers (B)+(C), or (B)+(C)+(B'), or (A)+(B)+(B')+(A') are separately produced and are subsequently joined to the other layers described in claim 1.

12. Packaging structure for biomedical films comprising the following overlapped layers, in the following order:
- (A) a laminate;
- (B) a supporting layer;
- (B') a supporting layer;
- (A') a laminate;
wherein:
- the laminates (A) and (A') are able to adhere to each other at room temperature and reversibly,
- the supporting layers are smaller in area than the laminates and are positioned fully within the laminate area, thereby determining a perimetral band P in which the internal faces of the laminates (A) and (A') are in reversible adhesive contact with each other.

13. Use of the structure according to claim 12, for the packaging of biomedical films.

14. Use according to claim 13, wherein said laminates, supporting layers and film have the features described in claims 1-12.

## Patentansprüche

1. Verpackungsstruktur für biomedizinische Filme umfassend die folgenden überlappenden Schichten in der folgenden Reihenfolge:
- (A) ein Laminat;
- (B) eine Trägerschicht;
- (C) einen biomedizinischen Film;
- (B') eine Trägerschicht;
- (A') ein Laminat;
wobei:
- die Laminate (A) und (A') bei Raumtemperatur reversibel aneinander haften
- die Trägerschichten geringere Ausmaße als die Laminate aufweisen und zur Gänze innerhalb der Laminatsfläche liegen, wodurch ein perimetrischer Streifen P bestimmt wird, innerhalb dessen die inneren Oberflächen der Laminate (A) und (A') miteinander in reversiblem adhäsiven Kontakt stehen.

2. Struktur gemäß Anspruch 1, wobei beide Laminate (A) und (A') an den entsprechenden inneren Oberflächen haftend sind.

3. Struktur gemäß Anspruch 1, wobei eines der beiden Laminate (A) und (A') auf seiner inneren Oberfläche haftend ist.

4. Struktur gemäß Ansprüchen 1-3, wobei die Laminate (A) und (A') mehrschichtige Strukturen sind.

5. Struktur gemäß Ansprüchen 1-4, wobei die Trägerschichten (B) und/oder (B') aus laminiertem Papier oder Polyester hergestellt sind.

6. Struktur gemäß Ansprüchen 1-5, wobei die Trägerschichten (B) und/oder (B') auf ihrer äußeren Oberfläche haftend sind.

7. Struktur gemäß Ansprüchen 1-6, wobei der biomedizinische Film ein Film zur oralen Anwendung ist.

8. Struktur gemäß Ansprüchen 1-7, in Form eine mehrfach verwendbaren Streifens enthaltend einen Stapel an oralen Filmen, die voneinander durch Markierungslinien getrennt sind.

9. Verfahren zur Herstellung einer Verpackungsstruktur für biomedizinische Filme, umfassend das Zusammenfügen der Komponenten (A), (B), (C), (B'), (A') wie in Anspruch 1 definiert und in der dort angegebenen Reihenfolge.

10. Verfahren nach Anspruch 9, umfassend die folgenden Schritte:
1. Bereitstellen eines ersten Laminatstreifens;
2. intermittierendes Aufbringen einer Trägerschicht auf dem in Schritt 1 erhaltenen Laminat;
3. Aufbringen eines Film auf der Trägerschicht, welche sich auf dem Laminat befindet, erhalten in Schritt 2;
4. Bereitstellen eines zweiten Laminatstreifens gemäß den Schritten 1 und 2;
5. Überlappen des in Schritt 3 erhaltenen Laminats mit demjenigen, das in Schritt 4 erhalten wurde;
6. Markieren und/oder Schneiden des in Schritt 5 erhaltenen Verbundlaminats in den Bereichen, welche jede Trägerschicht jeweils von der nächsten trennt.

11. Verfahren gemäß Anspruch 9, wobei die überlappten Schichten (B)+(C) oder (B)+(C)+(B') oder (A)+(B)+(B')+(A') separat hergestellt werden und anschließend mit den anderen, in Anspruch 1 beschriebenen Schichten verbunden werden.

12. Verpackungsstruktur für biomedizinische Filme umfassend die folgenden überlappten Schichten in der folgenden Reihenfolge:
- (A) ein Laminat;
- (B) eine Trägerschicht;
- (B') eine Trägerschicht;
- (A') ein Laminat;
wobei:
- die Laminate (A) und (A') bei Raumtemperatur reversibel aneinander haften können,
- die Trägerschichten geringere Ausmaße als die Laminate aufweisen und zur Gänze innerhalb der Laminatsfläche liegen, wodurch ein perimetrischer Streifen P bestimmt wird, innerhalb dessen die inneren Oberflächen der Laminate (A) und (A') miteinander in reversiblem adhäsiven Kontakt stehen.

13. Verwendung der Struktur gemäß Anspruch 12, für die Verpackung von biomedizinischen Filmen.

14. Verwendung gemäß Anspruch 13, wobei die Laminate, Trägerschichten und der Film die in den Ansprüchen 1-12 beschriebenen Merkmale aufweisen.

## Revendications

1. Structure d'emballage de films biomédicaux comprenant les couches chevauchantes suivantes, dans l'ordre suivant :
- (A) un stratifié ;
- (B) une couche de support ;
- (C) un film biomédical ;
- (B') une couche de support ;
- (A') un stratifié ;
dans laquelle :
- les stratifiés (A) et (A') adhèrent de façon réversible l'un à l'autre à température ambiante
- lesdites couches de support ont une aire plus faible que lesdits stratifiés et sont positionnés totalement dans la zone de stratifié, de manière à déterminer une bande périphérique P dans laquelle les faces internes des stratifiés (A) et (A') sont en contact adhésif réversible l'une avec l'autre.

2. Structure selon la revendication 1, dans laquelle les deux stratifiés (A) et (A') sont adhésifs sur les faces internes respectives.

3. Structure selon la revendication 1, dans laquelle un des deux stratifiés (A) ou (A') est adhésif sur sa face interne.

4. Structure selon les revendications 1 à 3, dans laquelle les stratifiés (A) et/ou (A') sont des structures multicouches.

5. Structure selon les revendications 1 à 4, dans laquelle les couches de support (B) et/ou (B') sont constituées de papier ou polyester stratifié.

6. Structure selon les revendications 1 à 5, dans laquelle les couches de support (B) et/ou (B') sont adhésives sur leur face externe.

7. Structure selon les revendications 1 à 6, dans laquelle le film biomédical est un film pour application orale.

8. Structure selon les revendications 1 à 7, sous la forme d'une bande multi-utilisation contenant une séquence de films oraux, séparés les uns des autres par des lignes de marquage.

9. Procédé de préparation d'une structure d'emballage pour films biomédicaux, comprenant l'assemblage des composants (A), (B), (C), (B'), (A') tels que définis dans la revendication 1 et dans l'ordre indiqué dans celle-ci.

10. Procédé selon la revendication 9, comprenant les étapes suivantes :
1. fourniture d'une bande stratifiée ;
2. application intermittente d'une couche de support sur le stratifié obtenu dans l'étape 1 ;
3. application d'un film sur la couche de support présente sur le stratifié obtenu dans l'étape 2 ;
4. fourniture d'une deuxième bande stratifiée selon les étapes 1 et 2 ;
5. chevauchement du stratifié obtenu dans l'étape 3 avec celui de l'étape 4 ;
6. marquage et/ou coupe du stratifié composite obtenu dans l'étape 5, dans les zones séparant chaque couche de support de la suivante.

11. Procédé selon la revendication 9, dans lequel les couches chevauchantes (B)+(C), ou (B)+(C)+(B'), ou (A)+(B)+(B')+(A') sont séparément produites et sont ensuite assemblées avec les autres couches décrites dans la revendication 1.

12. Structure d'emballage pour des films biomédicaux comprenant les couches chevauchantes suivantes, dans l'ordre suivant :
- (A) un stratifié ;
- (B) une couche de support ;
- (B') une couche de support ;
- (A') un stratifié ;
dans laquelle
les stratifiés (A) et (A') étant capables d'adhérer mutuellement à température ambiante et de façon réversible,
- les couches de support ont une aire plus petite que les stratifiés et sont positionnées totalement à l'intérieur de la zone de stratifié, de manière à déterminer une bande périphérique P dans laquelle les faces internes des stratifiés (A) et (A') sont en contact adhésif réversible l'une avec l'autre.

13. Utilisation de la structure selon la revendication 12, pour l'emballage de films biomédicaux.

14. Utilisation selon la revendication 13, dans laquelle lesdits stratifiés, couches de support et film possèdent les caractéristiques décrites dans les revendications 1 à 12.
